# EUROPEAN PATENT APPLICATION

(11) **EP 1 854 437 A1**
(43) Date of publication of application: **14.11.2007**
(21) Application number: 07380131.8
(22) Date of filing: 10.05.2007
(51) Int. Cl.: A61F 7/02

(54) **Personal device for producing heat or cold**

(30) Priority: 12.05.2006 ES 200601099 U
(71) Applicant: Melian del Castillo, Arturo, 38293 La Laguna Tenerife (ES)
(72) Inventor: Melian del Castillo, Arturo, 38293 La Laguna Tenerife (ES)
(74) Representative: Garcia-Cabrerizo y del Santo, Pedro Maria

(57) **Abstract**

Device for producing heat or cold at will and that can be carried on any part of the body, it is comprised by an aluminium plate (1), one or several Peltier cells (2), a thermal insulation (3), an on switch (5) and a change of state switch (6) which inverts the polarity of the Peltier cells (2), attached to the second face of the aluminium plate (1) through a plate (7) joined to the former plate by screws (9). This device has rechargeable batteries (4), placed on the second face of the aluminium plate, connected to the Peltier cells (2) through an on switch (5) and the change of state switch (6) and an elastic band (10) joined to the aluminium plate (1), by attachment means.

## Description

The present invention relates to a device that can be carried on any part of the body to supply heat or cold at will.

The device of the invention comprises an aluminium plate with thermal insulation on its entire surface except for an area where several Peltier cells are placed in cascade, such that each plate is in contact with the opposite pole of the following plate. The Peltier cells are supplied electrical power from rechargeable batteries through an on switch that allows regulating the power and a state change switch that allows inverting the polarity of the Peltier cells and thereby make the last Peltier cell, in contact with the human body, constitute the hot point or cold point of the assembly.

An elastic band joined to the aluminium plate provided with attachment means allows placing the device on various parts of the body.

This provides a pleasant sensation of heat or cold on the body.

To complement the above description and in order to aid a better understanding of the characteristics of the invention, a detailed description will be made of a preferred embodiment with reference to a set of drawings accompanying this descriptive memory where, for illustration purposes only and in a non-limiting sense the following is shown:
Figure 1 shows a plan view of the device of the invention.
Figure 2 shows a bottom view of the device of the invention.
Figure 3 shows a front elevation view of the device of the invention.
Figure 4 shows a rear view of the device of the invention.
Figure 5 shows a side view of the device of the invention.
Figure 6 shows a Peltier cell.
Figure 7 shows two Peltier cells in a cascade arrangement.
Figure 8 shows a Peltier cell in a specific state.
Figure 9 shows the Peltier cell of figure 8 after the change of state.
Figure 10 shows the wiring scheme of an arrangement with two Peltier cells in cascade.

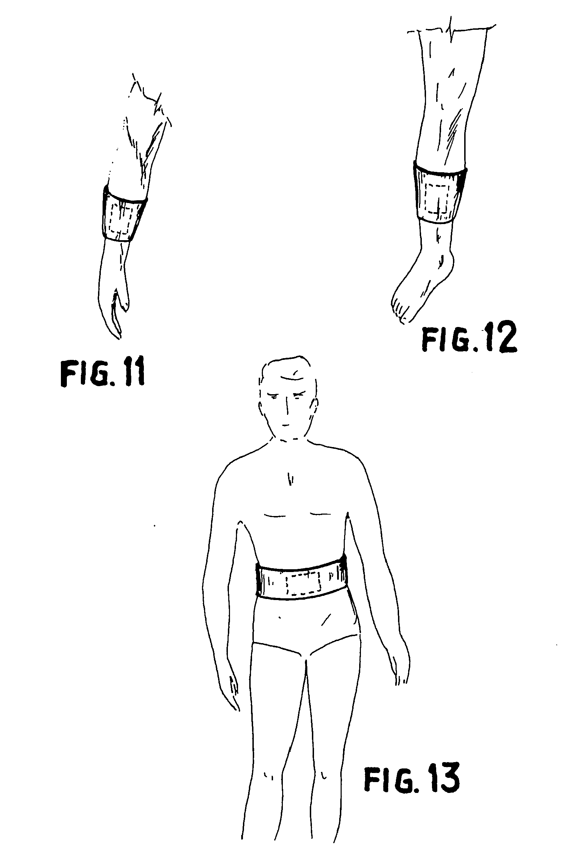

Figure 11 shows the device of the invention in its example of embodiment as a wrist band.
Figure 12 shows the device of the invention in its example of embodiment as an ankle strap.
Figure 13 shows the device of the invention in its example of embodiment as a lumbar belt.

In the aforementioned figures the numerical references correspond to the following elements:
1. Aluminium plate
2. Peltier cells
3. Thermal insulation
4. Rechargeable batteries
5. On switch
6. Change of state switch
7. Plate
8. Battery holder
9. Screws
10. Elastic band
11. Seams
12. Attachment means
13. Charger.

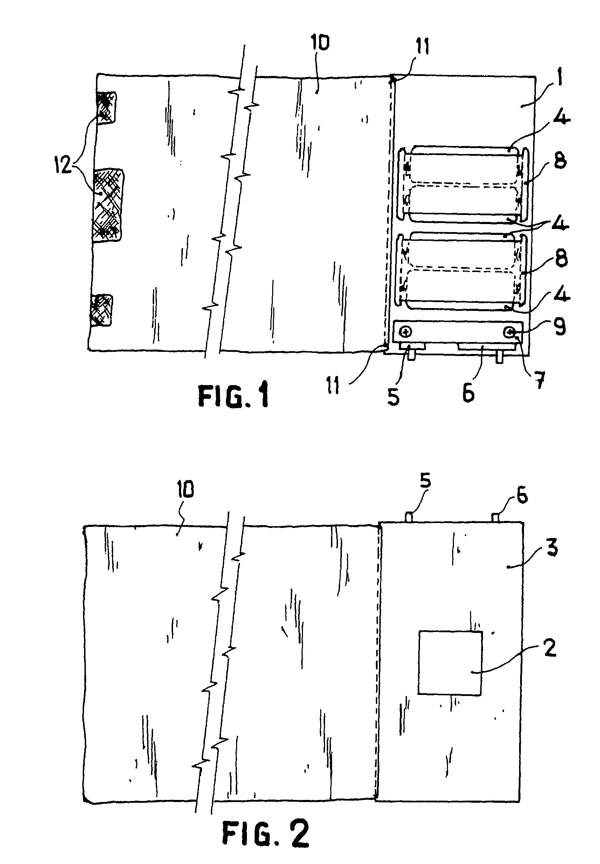

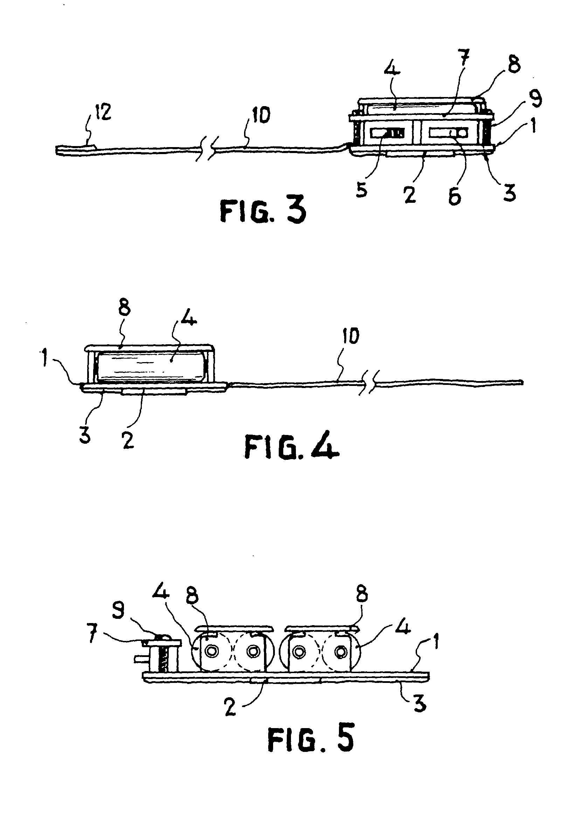

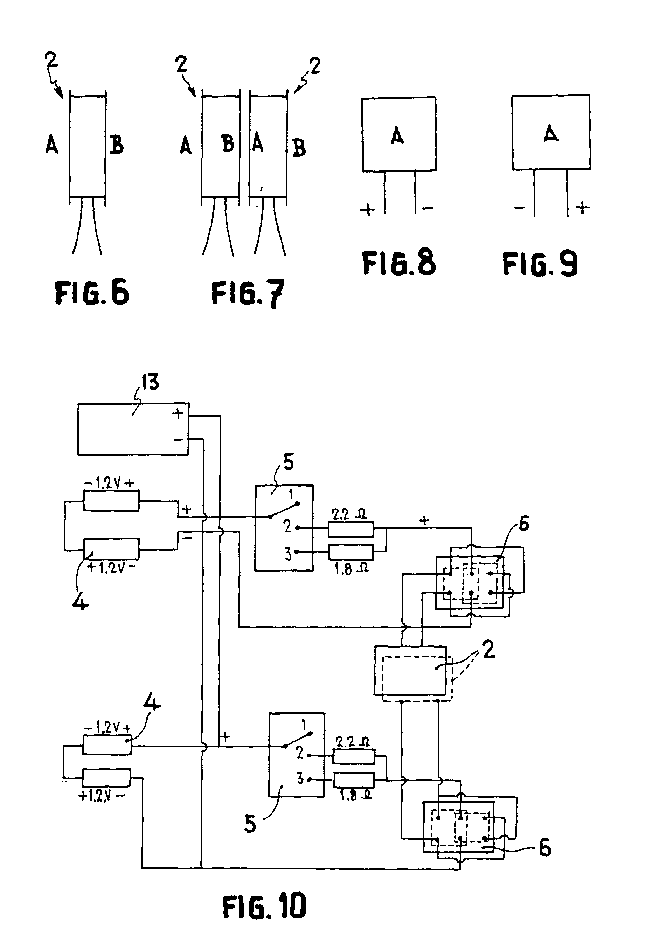

As can be seen in figures 1 to 5, the device of the invention comprises an aluminium plate (1) to which are attached, in a first face, a plurality of Peltier cells (2) disposed in cascade, that is, as shown in figure 7 with consecutive plates of different polarity in contact. A thermal insulation (3) covers this entire first face of the aluminium plate with the exception of the surface occupied by the Peltier cells (2).

The Peltier cells (2), disposed in cascade, are supplied electrical power by rechargeable batteries (4) through an on switch (5) and a change of state switch (6). Figure 10 shows an example of the wiring scheme for two Peltier cells (2) powered in parallel by two sets of two rechargeable batteries (4). In this case, four nickel metal hydride cells have been used with 1.2V and a 2,500 mAh capacity that can supply at least 1,000 charge - discharge cycles.

The on switch (5) and change of state switch (6) are attached to the aforementioned face of the aluminium plate (1) opposite that provided with the Peltier cells (2) by a plate (7) attached by screws (9). On the same face are provided a plurality of battery holders (8) meant to receive the rechargeable batteries (4).

An elastic band (10), advantageously an acrylic fabric, joined to the aluminium plate (1) by glue and/or several sewn seams (11) allows adjusting the device to the body such that it will not move by means of attachment means (12) which preferably consist of Velcro straps.

A charger (13) may be incorporated in the device of the invention, although it may also be an external element to save weight and bulk.

The operation of the device is as follows:

Peltier cells (2) are devices which when electrically powered provide a temperature difference across their end plates. When placed in cascade, as shown in figure 7, the total temperature difference is the sum of the temperature differences of each Peltier cell (2). On another hand, if one of its faces (face A, for example) is the hot point with a given polarity (figure 8) it will become the cold point if the polarity is inverted (figure 9).

This change of polarity is achieved in the device of the invention by a change of state switch (6).

The temperature difference is a function of the voltage with which the Peltier cells are powered and the electric current passing through them. Power regulation is obtained in the device of the invention by an on switch with three positions: a first position in which the power is shut off, a second position in which power is supplied through a 2.2 ohm resistance such that the unit operates at half-power, and a third position in which the power is supplied through a 1.8 ohm resistance such that the unit operates at full power.

On its part, the charger (13) will turn on when it is necessary to restore the power level of the rechargeable batteries (4).

The figures do not represent components such as connection wires which, although essential, are well known by an expert in the field as regards their use and arrangement.

## Claims

1. Personal device for producing heat or cold **characterized in that** it comprises:
- an aluminium plate (1);
- one or several Peltier cells (2) disposed in cascade on a first face of the aluminium plate;
- a thermal insulator (3) which covers the entire first face of the aluminium plate (1) except for the surface occupied by the Peltier cells;
- an on switch (5) and a change of state switch (6) that inverts the polarity of the Peltier cells (2), attached to the second face of the aluminum plate (1) through a plate (7) joined to the former plate by screws (9);
- rechargeable batteries (4) disposed on the second face of the aluminium plate connected to the Peltier cells (2) through the on switch (5) and the change of state switch (6);
- an elastic band (10) attached to the aluminium plate (1), provided with attachment means (12).

2. Personal device for producing heat or cold according to claim 1, **characterized in that** the on switch (5) has three positions: a first position in which power is cut off; a second position in which power is supplied through a first resistance; and a third position in which power is supplied through a second resistance with an ohm value lower than that of the first resistance.

3. Personal device for producing heat or cold according to claim 1, **characterized in that** it incorporates a charger (13) that can replenish the power level of the rechargeable batteries (4).

4. Personal device for producing heat or cold according to claim 1, **characterized in that** the elastic band (10) is an acrylic fabric.
